# EUROPÄISCHE PATENTSCHRIFT

(11) **EP 0 621 065 B1**
(45) Veröffentlichungstag und Bekanntmachung des Hinweises auf die Patenterteilung: **23.12.1998**
(21) Anmeldenummer: 94105875.2
(22) Anmeldetag: 15.04.1994
(51) Int. Cl.: B01D 9/00

(54) **Verfahren zur Trennung und Reinigung von Stoffen durch Schmelzkristallisation unter hohen Drücken**
Device for separating and purifying materials by melt crystallization under high pressure
Procédé pour la séparation et la purification de matériaux par cristallisation de fusion sous haute pression

(30) Priorität: 22.04.1993 DE 4313101
(43) Veröffentlichungstag der Anmeldung: 26.10.1994
(73) Patentinhaber: HOECHST AKTIENGESELLSCHAFT, 65929 Frankfurt am Main (DE)
(72) Erfinder: Steiner, Rudolf Prof. Dr., D-91056 Erlangen (DE); König, Axel Dr., D-70374 Stuttgart (DE); Rittner, Siegbert Dr., D-64546 Mörfelden (DE)

(56) Entgegenhaltungen:
- DE-A- 2 158 754
- DE-B- 2 135 539
- PATENT ABSTRACTS OF JAPAN vol. 13, no. 336 (C-623) (3684) 27. Juli 1989 & JP-A-01 115 404 (KOBE STEEL)

## Beschreibung

Unter Schmelzkristallisation versteht man ein Verfahren zur Trennung oder Reinigung von Stoffen, bei dem aus einer Schmelze eine oder mehrere Komponenten auskristallisiert und abgetrennt werden, während andere Bestandteile im nicht-erstarrten Teil der Schmelze, der sog. Restschmelze, verbleiben.

Eine solche Schmelzkristallisation unterscheidet sich von einer Lösungskristallisation dadurch, daß das zu trennende Stoffgemisch lediglich aufgeschmolzen, aber nicht in einem Hilfsmedium- dem Lösemittel- aufgelöst und daraus wieder auskristallisiert werden muß. Der Verzicht auf Zufügung eines Lösemittels - sei es Wasser oder ein organisches Medium - erbringt in vielen Fällen ökologische Vorteile: wo kein Wasser als Lösemittel eingesetzt wird, entsteht kein Abwasser, wo keine organischen Lösemittel benötigt werden, können sie keine Abluftbelastungen verursachen.

Mit den verschiedenen technischen Ausführungsformen der Schmelzkristallisation wird das Ziel verfolgt, bei der Trennung der Komponenten möglichst große Ausbeuten und hohe Reinheiten in angemessenen Kristallisationszeiten zu erreichen. Hierfür gibt es prinzipiell zwei Varianten:
- Verfahren, bei denen aus der Schmelze an gekühlten Flächen zusammenhängende Kristallschichten abgeschieden werden, so daß die restliche Flüssigkeit hiervon ohne weitere Trennoperation (allein durch die Schwerkraft) abfließen kann,
- Verfahren, bei denen die Schmelze durch Abkühlung in eine Kristallsuspension überführt wird, die in einem weiteren Verfahrensschritt (meist durch eine mechanische Flüssigkeitsabtennung) in Feststoff und Restschmelze getrennt wird.

Die Verfahren der ersten Gruppe arbeiten meist taktweise, sind apparativ einfach und betriebstechnisch sehr sicher. Hierzu gehören z.B. die schon seit vielen Jahrzehnten eingesetzten Rohrbündel-Kristallisatoren (Hoechst AG) oder die Rieselfilm-Kristallisatoren (Sulzer-MWB). Die Verfahren der zweiten Gruppe werden überwiegend kontinuierlich betrieben; bei manchen Ausführungen wird sogar ein Gegenstrom von Kristallen und Schmelze bewirkt. Allerdings sind hierfür komplizierte Apparate mit bewegten Einbauten erforderlich, auch die Betriebsführung ist wesentlich schwieriger. Zu dieser Gruppe gehören z.B. Kratzkühler (Brodie-Purifier) und Kristallisierkolonnen mit mechanischer Zwangsführung der sich bildenden Kristalle (Fa. Phillips, Fa. Kureha).

Bei Schmelzkristallisationsverfahren können sehr hohe Reinheiten erzielt werden, insbesondere wenn man eine Betriebsweise anwendet, die durch folgende Teilschritte gekennzeichnet ist: Zunächst wird ein Teil der Schmelze durch Kühlung auskristallisiert, danach wird die Restschmelze vom Kristallisat abgetrennt und letzteres unter langsamer Temperaturerhöhung wieder etwas angeschmolzen, um noch anhaftende oder zwischen den Kristallen eingeschlossene Restschmelze abzutrennen, bevor in einem letzten Schritt das so gereinigte Kristallisat als ganzes wieder aufgeschmolzen wird. Den Zwischenschritt des Entfernens anhaftender oder eingeschlossener Verunreinigungen bezeichnet man im betrieblichen Sprachgebrauch als "Schwitzen" oder "Abtropfen".

Auch hochkonzentrierte Lösungen mit einem vergleichsweise geringen Lösemittelanteil können nach diesem Verfahrensprinzip gereinigt werden, weil sie in ihrem Erstarrungsverhalten Schmelzen nahe kommen.

Üblicherweise werden Schmelzkristallisationen unter Normaldruck ausgeführt, allenfalls wird zur Förderung der Schmelze ein etwas erhöhter Druck benötigt. Für die Anwendung höherer Drücke zur Verbesserung der Trennwirkung bei der Schmelzkristallisation sind bisher nur wenige Vorschläge und Versuche bekannt geworden.

Ein Verfahrensvorschlag stammt von einer japanischen Forschungsgruppe (Industrial Crystallization 84, edited by S. J. Jancic and E. J. de Jong, Elsevier Science Publishers B. V., Amsterdam, 1984) und beruht darauf, daß die eigentliche Kristallisation unter hohen Drücken durchgeführt wird und anschließend eine Reinigung des Kristallisats durch partielles Wiederaufschmelzen infolge einer Druckabsenkung stattfindet. Ãhnliche Verfahren sind aus der JP-A-0 111 5404, der JP-A-54 06 2977 und der JP-A-54 05 2678 bekannt.

Die Schrift DE-A-2 135 539 offenbart ein Verfahren zur Trennung oder Reinigung von Stoffen durch Kristallisation aus hochkonzentrierten Lösungen unter erhöhten Drücken von über 100 bar, wobei nach der Kristallisation der Druck allmählich wieder abgesenkt und das hochreine Kristallisat, zwischen dessen Kristallen dann fast keine Flüssigkeit und kein Gas eingeschlossen ist, von der bei der Druckabsenkung wieder entstehenden, die Verunreinigungen aufnehmenden flüssigen Phase getrennt wird. Der hohe Druck wird durch Volumenveränderung des die hochkonzentrierte Lösung beinhaltenden Behälters aber auch durch Aufpressen eines Fluids erzeugt. Es ist jedoch in der Schrift (1) nicht die Rede davon, in der kristallisierenden Lösung ein Gas zu lösen, welches nach dem Kristallisieren beim Entspannen wieder ausgast.

Der Schwitzvorgang, der üblicherweise durch eine langsame Temperaturerhöhung nach der Kristallisation bewirkt wird, wird bei dieser Verfahrensvariante durch eine Druckabsenkung erzielt. Aus den Publikationen geht hervor, daß der Druck bei diesem Verfahren mechanisch durch Einpressen eines Stempels in den Kristallisierbehälter erzeugt wird. In einer entsprechenden Versuchsanlage, die mit einem 1,5 l-Autoklaven ausgestattet ist, wurden z.B. Mischungen p- und m-Kresol kristallisiert und getrennt, wobei die Kristallisation bei etwa 1500 bar ablief, während in der Reinigungsphase der Druck kontinuierlich bis auf Normaldruck abgesenkt wurde. Die Schwachstelle bei diesem Verfahren ist das Filtrationsorgan zur Abtrennung der flüssigen von der kristallinen Phase. Die schnell ablaufende Kristallisation führt zu kleinen, schlecht ausgebildeten Kristallen, die bekanntlich jede Feststoff-Flüssigkeits-Abtrennung äußerst erschweren. Da dieses Problem bisher noch nicht befriedigend gelöst werden konnte, ist eine technische Anwendung dieses Verfahrens bisher unterblieben.

Ein anderer Verfahrensvorschlag stammt von einem finnischen Forscherteam (Silventoinen, J. Crystal. Growth. 66 (1984) 179). Hierbei soll die Verschiebung des eutektischen Punktes einer Schmelze mit steigendem Druck ausgenutzt werden, und zwar in der Weise, daß eine Schmelze, deren Ausgangzusammensetzung zwischen den eutektischen Punkten für zwei Druckstufen liegt, abwechselnd beim höheren und niedrigeren Druck kristallisiert wird. Hierdurch können theoretisch beide Komponenten einer aus zwei Stoffen bestehenden Schmelze mit relativ hoher Reinheit gewonnen werden. Dafür bedarf es aber einer erheblichen Abhängigkeit der eutektischen Zusammensetzung vom Druck, wie sie nur bei wenigen Systemen gegeben ist. Eine technische Realisierung dieses Verfahrensvorschlag ist bisher ebenfalls nicht bekannt geworden.

Es wurden nun ein Verfahren zur Durchführung der Schmelzkristallisation entwickelt, das es gestattet, durch Anwendung erhöhter Drücke besonders gute Trenn- und Reinigungseffekte zu erzielen.

Die Aufgabe wird durch ein Verfahren zum Trennen und Reinigen von Stoffen durch Schmelzkristallisation unter Druck gelöst, bei dem
a) in der zu kristallisierenden Schmelze Inertgas bei Gasdrücken von 50 bis 5000 bar gelost wird,
b) die Schmelze bei Drucken 50 bis 5000 bar kristallisiert wird,
c) das nach der Kristallisation bei einer Druckabsenkung wieder ausgast, wodurch noch anhaftende oder eingeschlossene Verunreinigungen entfernt werden.

Die Auflösung des Gases in der Schmelze kann durch mechanische Vorgänge, wie z.B. Bewegen der Schmelze oder Einrühren bzw. Eindüsen des Gases, verbessert werden. Die Druckabsenkung nach der Kristallisation kann stufenweise vorgenommen werden. Als Gase kommen solche in Betracht, die in der jeweiligen Schmelze löslich sind. Für die Stoffsysteme Naphthalin/Biphenyl und p-/m-Dichlorbenzol eignen sich z.B. Stickstoff, Kohlendioxid, Ethan, Ethen, Propan etc. oder deren Gemische. Zwecks besserer Löslichkeit kann das Gas unter Bewegen der Schmelze über Düsen und dergleichen in die Schmelze verteilt werden. Die Schmelze kann bei Drücken von 50 bis 5000 bar bei entsprechender Temperaturabsenkung kristallisiert werden. Der erforderliche Druck hängt vom Stoffsystem und der Zusammensetzung der Komponenten ab.

Die Vorteile des Verfahrens sind im wesentlichen im Entgasen des Kristallisats zu sehen, durch das das Kristallisat aufgelockert wird, was den Schwitzprozeß wirkungsvoll verbessert. Die stufenweise Druckentspannung erlaubt Schwitzöle mit unterschiedlichem Verunreinigungsgrad abzutrennen.

Während bei dem oben erwähnten japanischen Verfahrensvorschlag der Aufbau des Druckes und auch das Abpressen der Restschmelze durch Einfahren eines Stemples in den Kristallisierapparat, also mechanisch, erfolgt, erweist es sich als viel günstiger, den Druck durch Aufpressen von Gas zu erzeugen oder die Schmelze in einen unter Druck stehenden Kristallisator einzupumpen. Dabei kann es abhängig vom Stoffsystem für die Kristallisation auch von Vorteil sein, den Druck allmählich oder schrittweise auf den Enddruck zu steigern. Durch geeignete Maßnahmen sollte sicher gestellt werden, daß sich dabei möglichst viel Gas in der Schmelze löst. Dies führt dazu, daß bei der nachfolgenden Entspannung das Kristallisat nicht nur oberflächlich wiederaufschmilzt, sondern durch das Entgasen der gesamte Kristallverband aufgelockert wird, was eine Entfernung von eingeschlossenen Verunreinigungen überhaupt erst ermöglicht.

Bei der Auswahl des Gases ist dessen Löslichkeit in der Schmelze von ausschlaggebender Bedeutung. Bei dem hier vorgeschlagenen Verfahren sind z.B. Stickstoff und Kohlendioxid, aber auch niederere Kohlenwasserstoffe, wie Ethan, Ethen, Propan u.ä. wegen ihrer günstigen physikalischen Eigenschaften meist gut geeignet. Selbstverständlich können auch Gasgemische verwendet werden, sofern sie miteinander nicht reagieren. Die angewandten Drücke können je nach Stoffsystem und Zusammensetzung der Komponenten Werte bis zu 5000 bar erreichen. Hohe Drücke werden immer dann besonders von Vorteil sein, wenn der Schmelzpunkt des gewünschten Produkts bei niedrigeren Drücken tief, d.h. unter 0°C liegt, oder wenn die Lage des Eutektikums für das zu reinigende Produkt ungünstig ist und verändert werden soll.

Die Druckabsenkung kann nach der Kristallisation und Restschmelzabtrennung ein- oder mehrstufig durchgeführt werden, wobei die stufenweise Druckentspannung eine Abnahme verschieden stark verunreinigter Restschmelzen erlaubt, wobei die relativ sauberen Fraktionen gegebenenfalls im Verfahren rezirkuliert werden können.

Apparate, in der die erfindungsgemäße Schmelzkristallisation durchgeführt werden kann, können auf verschiedene Weise konstruiert sein. So kann im einfachsten Falle bereits in einem Druckautoklaven bzw. Platten- oder Röhrenwärmetauscher, der mit dem entsprechenden Inertgas unter Druck steht, die Schmelze ohne oder mit Kühlung kristallisiert werden. Ebenso geeignet ist ein Rieselfilmkristallisator, wie er z.B. von der Firma Sulzer, Winterthur, beschrieben wird. In einem solchen Apparat bildet sich häufig eine an Gaseinschüssen reiche, porige Kristallschicht, die bei der Entspannung einen wirkungsvollen Kristallschwitzprozeß erfährt.

Insgesamt führt diese Art der Schmelzkristallisation durch den Entgasungsvorgang zu erstaunlich guten Reinigungseffekten, was für die Herstellung von hochreinen Substanzen einen erheblichen Fortschritt bedeutet.

### Beispiele:

Um die Wirksamkeit des vorgeschlagenen Verfahrens zu belegen, wurden Schmelzkristallisationsversuche unter Druck und Inertgaszusatz in zwei verschiedenen Apparaturen und an zwei Stoffsystemen durchgeführt.

Bei der einen Apparatur handelt es sich um einen Autoklaven von ca. 1,5 l Inhalt, der mit zwei gegenüberliegenden schlitzförmigen Sichtfenstern versehen und für einen maximalen Betriebsdruck von 300 bar und eine Betriebstemperatur von 250°C ausgelegt ist. In dem Autoklaven wurde ein Reagenzglas freischwebend aufgehängt, in das das Ausgangsgemisch eingefüllt wird. In der Nähe der zu untersuchenden Probe wird die Temperatur mit einem Thermoelement gemessen. Die Temperaturregelung erfolgt über die Heizung des Autoklaven mittels eines Flüssigkeitsthermostaten.

Die andere Apparatur besteht aus einem Autoklaven von 0,2 l Inhalt mit einer innen angeordneten Wärmetauscherplatte. Dieser Autoklav wurde so auf einem Gestell montiert, daß er während des Versuchs um die Gestellachse gedreht werden kann. Er wird über einen Heizmantel elektrisch beheizt, während die Wärmetauscherplatte an einen Flüssigkeitsthermostaten angeschlossen ist. In Kristallisierstellung befindet sich die Platte im unteren Teil und ist von Schmelze umgeben. Nach Beendigung der Kristallisation wird der Autoklav gekippt, so daß die Platte in den oberen Teil gelangt und die Restschmelze und danach - während der Druckabsenkung - die Schwitzölfraktionen ablaufen können.

Als Stoffsysteme wurden Naphthalin/Biphenyl und p-/m-Dichlorbenzol verwendet.

### Beispiel 1

Mit diesem Versuch, der im Sichtfensterautoklaven ausgeführt wurde, wurden etwa 10 g eines Gemisches aus 66 % Naphthalin und 34 % Biphenyl eingesetzt, das unter einem Stickstoffdruck von 150 bar bei Temperaturen oberhalb 61°C schmelzflüssig vorlag. Bei der Abkühlung zeigten sich bei 60,4°C erst Kristalle, bei 59,8°C war etwa 1/6, bei 58,3°C etwa 9/10 des Volumens mit Kristallen durchsetzt, bei 54°C war alles "fest", d.h. es war keine Restschmelze mehr zu erkennen. Wurde der Druck dann stufenweise herabgesetzt, zeigten sich bei ca. 100 bar die ersten Gasblasen, bei 65 bar wurden die Kristalle trüb, bei 30 bar bildete sich eine flüssige Phase (wobei die Temperatur inzwischen auf 49,5°c gefallen war) und bei 1 bar (und 38,7°C) erfüllte die flüssige Schicht schließlich etwa 1/5 des Volumens. Diese Restschmelze begann bei weiterer Abkühlung erst unterhalb 38°C langsam zu kristallisieren.

Eine anschließend ausgeführte Analyse zeigte deutliche Unterschiede in den Zusammensetzungen der beiden Phasen: das Kristallisat enthielt etwa 75 %, die separierte Restschmelze nur 46 % Naphthalin.

Dieser Trenneffekt ist in Anbetracht der sehr einfachen Versuchsdurchführung (Lösung des Gases in ruhender Schmelze; Trennung von Kristallisat und Restschmelze nur durch Absetzen) erstaunlich hoch.

Ein bei Normaldruck ausgeführter Referenzversuch ergab, daß die Kristallisation der Ausgansgschmelze hierbei erst bei 57,0°C beginnt und nach Abkühlen der Temperatur auf Werte unterhalb 50°C keine Abtrennung einer Restschmelze mehr möglich ist.

### Beispiel 2

Dieser Versuch wurde im Autoklaven mit Wärmetauscherplatte ausgeführt. In den Autoklaven wurden 50 g eines Gemisches mit 70 % Naphthalin und 30 % Biphenyl eingefüllt, der Deckel mit der daran befestigten Wärmetauscherplatte aufgesetzt und fest verschlossen und der Autoklav dann gedreht, so daß sich die Platte im unteren Teil befand. Nach Aufdrücken von Stickstoff auf 100 bar wurde der Autoklaveninhalt auf 75°C aufgeheizt; die Wärmetauscherplatte wurde auf die gleiche Temperatur eingeregelt. Bei diesen Bedingungen wurde die Schmelze 1 h lang temperiert und unter Bewegen des Autoklaven mit Gas gesättigt. Dann wurde die Temperatur des Wärmetauschers auf 50°C und 1 h später auch die Autoklaventemperatur auf diesen Wert gesenkt. Nach einer weiteren Stunde wurde die Apparatur wieder gedreht, so daß sich die Wärmetauscherplatte nun oben befand und die Restschmelze ablaufen konnte. Dann wurde der Druck in Intervallen von 10 bar/5 min bis auf Normaldruck abgesenkt, um eingeschlossene Anteile an Restschmelze und Schwitzöle aus dem Kristallverbund herauszuholen. Anschließend wurde der Autoklav auf Raumtemperatur abgekühlt und geöffnet.

Dabei zeigte sich, daß die Wärmetauscherplatte von einem Kristallisat umgeben ist, dessen mittlere Zusammensetzung etwa 83 % Naphthalin beträgt. Eine genaue Untersuchung ergab, daß der Naphthalin-Gehalt im Inneren der Kristallschicht (mit bis zu 89 %) deutlich höher ist als in der Außenschicht (mit etwa 73 %). Diese Differenz ist erklärbar, wenn die Restschmelze tatsächlich beim Entspannen aus dem Kristallverbund herausgesaugt wird: dann ist der geringere Naphthalin-Gehalt sehr wahrscheinlich durch noch nicht gänzlich abgetropfte Restschmelze verursacht. Am Boden des Autoklaven hatte sich eine Schicht angesammelt, die eine Zusammensetzung von etwa 50 % Naphthalin aufwies.

Bei einem Referenzversuch unter Normaldruck sammelt sich am Boden des Druckgefäßes hingegen keine Restschmelze an.

So konnte auch durch diesen Versuch die gute Trennwirkung dieser Verfahrensvariante und insbesondere der Einfluß der Entgasung auf den Trennvorgang belegt werden.

### Beispiel 3

Bei einem weiteren Versuch, der ebenfalls im Autoklaven mit der Wärmetauscherplatte durchgeführt wurde, wurden 68 g eines Gemisches aus 88 % p-Dichlorbenzol und 12 % m-Dichlorbenzol eingesetzt. Dieses Gemisch wurde unter einem Stickstoffdruck von 100 bar kristallisiert. Die Ausgangstemperatur lag bei 65°C (Schmelze), der Kristallisationsbeginn bei 48°C, das Kristallisationsende bei 30°C. Nach der anschließenden Druckentspannung wurden ca. 48 g Kristallisat mit einer Reinheit von 99,95 % p-Dichlorbenzol vorgefunden, während die Restschmelze etwa 62 % p-Dichlorbenzol enthielt.

Zwei Referenzversuche bei Normaldruck erbrachten zwar auch noch eine Trennung in Kristallisat und eine Restschmelze, jedoch lag die Reinheit des Kristallisats mit 99,41 % bzw. 99,63 % immer niedriger als bei dem Druckversuch mit anschließender Endgasung. Der Unterschied in der Qualität der Endprodukte wird noch augenfälliger, wenn man die Quantitäten der Verunreinigungen (ohne Entgasung: 0,59 bzw. 0,37 %; mit Entgasung nur 0,05 %) gegenüberstellt: bei der Verfahrensweise mit Druckentspannung ist die Reinheit nahezu eine Zehnerpotenz höher.

### Beispiel 4

Bei einem weiteren Versuch, diesmal mit einem anorganischen Stoff, der ebenfalls im Autoklaven mit Wärmetauscherplatte durchgeführt wurde, sind 60 g eines Gemisches eingesetzt worden, welches aus 94 % phosphoriger Säure und 6 % verschiedener geradkettiger Fettsäuren mit einer Kohlenstoffkettenlänge von C₁₂ bis C₁₆ bestand. Dieses Gemisch wurde unter einem Stickstoffstrom von 50 bar kristallisiert. Die Ausgangstemperatur lag bei 69°C (Schmelze), der Kristallisationsbeginn bei 60°C, das Kristallisationsende bei 40°C. Nach der anschließenden Druckentspannung wurden 44 g Kristallisat mit einer Reinheit von 99,6 % phosphoriger Säure gewonnen. Ein Referenzversuch bei Normaldruck erbrachte zwar auch eine Trennung in Kristallisat und Restschmelze, jedoch lag die Reinheit des Kristallisats mit 97,7 % wesentlich niedriger als beim Druckversuch mit anschließender Endgasung.

Dies belegt noch einmal die Wirksamkeit des erfindungsgemäßen Verfahrens.

## Patentansprüche

1. Verfahren zur Trennung oder Reinigung von Stoffen durch Kristallisation aus Schmelzen oder hochkonzentrierten Lösungen unter erhöhten Drücken wobei
a) in der kristallisierenden Schmelze Inertgas bei Gasdrücken von 50 bis 5000 bar gelöst wird,
b) die Schmelze bei Drücken von 50 bis 5000 bar kristallisiert wird und
c) das Gas nach der Kristallisation bei einer Druckabsenkung wieder ausgast, wodurch noch anhaftende oder eingeschlossene Verunreinigungen entfernt werden.

2. Verfahren nach Anspruch 1, dadurch gekennzeichnet, daß die Schmelze unter Stickstoff-, Kohlendioxid-, Ethan-, Ethen-, Propan-Atmosphäre oder einer Atmosphäre aus deren Gemische unter Druck gesetzt wird.

## Claims

1. A process for separating and purifying substances by crystallization from melts or highly concentrated solutions under high pressures, wherein
a) inert gas is dissolved in the crystallizing melt at gas pressures from 50 to 5000 bar
b) the melt is crystallized at pressures of 50 to 5,000 bar and
c) the gas is expelled again after the crystallization with reduction in the pressure, with the result that still adhering or occluded impurities are removed.

2. The process as claimed in claim 1, wherein the melt is subjected to pressure under a nitrogen, carbon dioxide, ethane, ethene or propane atmosphere or an atmosphere comprising mixtures thereof.

## Revendications

1. Procédé pour la séparation et la purification de matières par cristallisation en fusion ou d'une solution hautement concentrée sous de pression élevée, où :
a) on dissout un gaz inerte à une pression de 50 à 5000 bars dans la masse fondue à cristalliser,
b) on cristallise la masse fondue sous une pression de 50 à 5 000 bars, et
c) on évacue le gaz après la cristallisation par diminution de la pression en éliminant les impuretés encore adhérentes ou renfermées.

2. Procédé selon la revendication 1, caractérisé en ce que on met la masse fondue sous pression sous une atmosphjère d'azote, de dioxyde de carbone, d'éthane, d'éthène, de propane ou de leurs mélanges mélanges.
